# EUROPEAN PATENT SPECIFICATION

(11) **EP 3 962 413 B1**
(45) Date of publication and mention of the grant of the patent: **08.04.2026**
(21) Application number: 20802489.3
(22) Date of filing: 30.04.2020
(51) Int. Cl.: A61F 2/16, C08F 220/18, A61L 27/16, C08F 220/30, G02B 1/04, C08F 2/48

(54) **A NEW ADVANCED FORMULATION FOR HYDROPHOBIC INTRAOCULAR LENSES AND THEIR PRODUCTION METHOD**
NEUE FORTSCHRITTLICHE FORMULIERUNG FÜR HYDROPHOBE INTRAOKULARLINSEN UND DEREN HERSTELLUNGSVERFAHREN
NOUVELLE FORMULATION AVANCÉE POUR LENTILLES INTRAOCULAIRES HYDROPHOBES ET MÉTHODE DE PRODUCTION ASSOCIÉE

(30) Priority: 03.05.2019 TR 201906605
(43) Date of publication of application: 09.03.2022
(73) Proprietor: VSY Biyoteknoloji Ve Ilac Sanayi Anonim Sirketi, Istanbul (TR)
(72) Inventor: MURAT, Oktay, Istanbul (TR)
(74) Representative: Sevinç, Cenk
(86) International application number: PCT/TR2020/050362
(87) International publication number: WO 2020/226588

(56) References cited:
- WO-A1-2016/201351
- WO-A1-2016/201351
- WO-A2-2006/126095
- JP-A- H08 299 373
- US-A- 5 814 680
- US-A1- 2003 193 100
- US-A1- 2008 139 769
- US-A1- 2008 306 587
- US-A1- 2010 211 170
- US-A1- 2011 037 184
- US-A1- 2018 153 682

## Description

### Field of the Invention

The present invention relates to a new advanced formulation for hydrophobic intraocular lens and their use for cataract disease

### Description of the Related Art

Cataracts are a major cause of blindness in the world and the most prevalent ocular disease. When the disability from cataracts affects or alters an individual's activities of daily living, surgical lens removal with intraocular lens (IOL) implantation is the preferred method of treating the related visual limitations. A cataract is defined as an opacity of a patient's lens, whether it is a localized opacity or a diffuse general loss of transparency. To be clinically significant, however, the cataract must cause a significant reduction in visual acuity or a functional impairment. A cataract occurs as a result of aging or secondary to hereditary factors, trauma, inflammation, metabolic or nutritional disorders, or radiation. Age related cataract conditions are the most common.

In treating a cataract, the surgeon removes the crystalline lens matrix from the lens capsule and replaces it with an intraocular lens ("IOL") implant. The typical IOL provides a selected focal length that allows the patient to have fairly good distance vision. It is desirable to provide an ophthalmic device, such as an IOL, in which the polymer have properties that would allow it to be deformed to a delivery configuration to enable its implantation in the eye, yet return to a pre-implantation configuration after being implanted in the eye. In addition, it would be desirable that the polymeric composition have a sufficient high refractive index. The polymeric compositions described herein can be used in an IOL, such as any of the hydrophobic IOLs described in U.S. Patent Application US5814680, US 6241766, US 7947796, US 85 57 89 US 2011/037184, US 2018/153682, US 2003/193100, US 2008/306587, JPH-08299373, US 2008/139769 and US 9907 881.

In the above mentioned Patents the polymeric materials does not combine the desired properties, like less adhesive properties, high refractive index, and the material property returning to their initial configuration once implanted in the eye in a proper period time, approximately 10 to 20 seconds.

For implantable devices that must be implanted through an incision in the sclera, it is generally desirable that the incision in the sclera be as small as possible while still being able to deform the device without damaging it. The device must also be able to reform to its initial configuration after delivery.

### Summary of the Invention

The inventive polymer described herein can be used in ophthalmic device that need to be deformed to be delivered through an incision, yet will return to their initial configuration once implanted in the eye.

### Detailed Description of the Invention

One aspect of the invention is a formulation developed for hydrophobic intraocular lenses, which can be used in the ophthalmic device inserted into the eye through a cut in the treatment of cataracts, it comprises alkyl acrylate, fluoro acrylate and phenyl methacrylate characterized in that it comprises by mass; 46.0% phenylethyl methacrylate (PEMA), 40.0% butyl acrylate (BA), 10.0% trifluoroethyl methacrylate (TFEMA), 3.0% Ethyleneglycoldimethacrylate (EGDMA) as a crosslinker, 0.5% of 2- (2'-Hydroxy- 5'-methacryloxy ethylphenyl) -2H-benzotriazole (UV absorber) and 0.5% azobisisobutyronitrile (AIBN).

One aspect of the disclosure is a polymeric material for an ophthalmic device wherein the material includes an alkyl acrylate, a fluoroacrylate, and a phenyl acrylate, wherein there is an effective amount of 5% to 15% of the fluoroacrylate to provide the polymer material with less adhesive properties.

In some embodiments of the disclosure there is an effective amount of 30% to 50% of the alkyl acrylate to provide the polymer material with a modulus of elasticity between about 0.1 Mpa and about 0.6 Mpa. In some embodiments there is an effective amount of 40% to 50% of the phenyl acrylate to provide the polymeric composition with a refractive index between about 1.48 and about 1.54.

In the present invention, the cross-linking agent is ethylene glycol dimethylacrylate. The ultraviolet light absorbing material is 2-(2'-Hydroxy- 5'-methacryloxy ethylphenyl) -2H-benzotriazole.

The polymeric material can be used for an intraocular lens which comprises a central optic portion and a peripheral non-optic portion. In some embodiments the central optic portion is made at least in part of the polymeric material, whereas the non-optic portion is comprised of a second polymeric material different than the polymeric material in the central optic portion. In some embodiments the central optic portion and peripheral non-optic portion are made from substantially the same polymeric material.

The following inventive formulation is added together and mixed well:

**Table 1. Example**

| **Monomer** | **Amount [%]** | **Quantity [g]** |
|---|---|---|
| Phenyl ethyl methacrylate (PEMA) | 46,0 % | 23,0 g |
| Butyl acrylate (BA) | 40,0 % | 20,0 g |
| Trifluor ethylmeth acrylate (TFEMA) | 10,0 % | 5,0 g |
| Ethyleneglycoldimethacrylate (EGDMA) | 3,0 % | 1,5 g |
| 2-(2'-Hydroxy-5'-methacryloxyethylphenyl)-2H-benzotriazole (UV absorber) | 0,5 % | 0,25 g |
| Azo isobutyronitrile (AIBN) | 0,5 % | 0,25 g |

Pour the formulation into a two-part mold cavity made of polypropylene. Seal the mold with no air entrapment.

Polymer curing is carried out by placing the sealed mold inside a chamber equipped with UV lamps. Polymerization can be carried out in one hour or less depending on the lamp intensity. Optional polymerization could be a thermal polymerization with different temperature steps for about 24 hours. Postcuring can be carried out in thermal oven at 90 C to 100 C for 1-2 hours to ensure more complete polymerization. The resulting polymer has a refractive index of 1.51.

When IOL solid components were manufactured from BA-TFEMA-PEMA-EGDMA compositions, the polymer have the fiollowing properties: biocompatible, optically clear, high refractive index and desirable levels of mechanical strength. These components also exhibited a level of flexibility and elastic memory sufficient to permit the IOL to be inserted without breaking through a small incision in the cornea, and later capable of regaining its original shape within an acceptable amount of time.

The present invention relates generally to improved polymeric materials. The polymeric materials have less adhesive properties, relatively high refractive indices, and are adapted to assume an initial configuration after being deformed during implantation in the human body. While the polymeric materials can be used in a wide variety of applications, the polymers are described herein in their use in an ophthalmic device such as an intraocular lens ("IOL"). In addition to an IOL, the polymeric compositions of the present invention can also be used in other ophthalmic devices such as, but not limited to, contact lenses, keratoprostheses, capsular bag extension rings, corneal inlays, corneal rings, or other ophthalmic devices.

For implantable devices that must be implanted through an incision in the sclera, it is generally desirable that the incision in the sclera be as small as possible while still being able to deform the device without damaging it. The device must also be able to reform to its initial configuration after delivery. The inventive polymers described herein can therefore be used in ophthalmic device that need to be deformed to be delivered through an incision, yet will return to their initial configuration once implanted in the eye. In addition, it may be desirable to increase the refractive index ("RI") of the ophthalmic device to increase its refractory power. An increase in the RI of the bulk polymer can allow the device to be thinner, yet maintain a desired power. This can also provide the device with a smaller delivery profile to reduce the size of the incision in the eye during implantation.

One embodiment of the invention is a polymeric material for an ophthalmic device. In a preferred embodiment, the composition comprises butyl acrylate, trifluoroethyl methacrylate, phenylethyl methacrylate, and ethylene glycol dimethacrylate as a cross-linker. To achieve the desired properties of the polymer described above, the above mentioned particular monomers have to be selected in combination.

### Reference Examples

A number of experiments were carried out to achieve compositions with desired properties. In a first experiment solid lens components were produced comprising a copolymer of Butyl acrylate (BA) and Trifluoroethyl methacrylate (TFEMA). Adding TFEMA in from about 5% to about 15% leads to a polymer material with less adhesive properties.

In a second experiment, phenylethyl methacrylate (PEMA) was copolymerized together with the BA-TFEMA composition from above. A sharp increase in the refractive index of this composition was observed, particularly when BA was present from about 30% to about 50%, when PEMA was present from about 35% to about 55%, and when TFEMA was present from about 5% to about 15%. More specifically, an increase of the refractive index to 1.51 was obtained, with an elastic modulus measured in the 0.1-0.5 Mpa range.

In a third experiment, a cross-linking agent was added to the BA-PEMA-TFEMA composition as an additional constituent. In particular, ethylene glycol dimethacrylate was found to operate as a suitable cross-linking agent with this composition. The foregoing cross-linking agents may be added in amounts of less than about 5% of the BA-TFEMA-PEMA composition and cause the composition polymers to become interlaced within a tri-dimensional space, providing for a compact molecular structure having an improved elastic memory over the non-crosslinked composition.

## Claims

1. A formulation developed for hydrophobic intraocular lenses, which can be used in the ophthalmic device inserted into the eye through a cut in the treatment of cataracts, it comprises alkyl acrylate, fluoro acrylate and phenyl methacrylate **characterized in that** it comprises by mass; 46.0% phenylethyl methacrylate (PEMA), 40.0% butyl acrylate (BA), 10.0% trifluoroethyl methacrylate (TFEMA), 3.0% Ethyleneglycoldimethacrylate (EGDMA) as a crosslinker, 0.5% of 2- (2'-Hydroxy- 5'-methacryloxy ethylphenyl) -2H-benzotriazole (UV absorber) and 0.5% azobisisobutyronitrile (AIBN).

2. Use of the formulation developed for hydrophobic intraocular lenses according to claim 1, in intraocular lenses whose central optic portion is made of a polymeric material and whose peripheral non-optic portion is made of a second polymeric material different from the polymeric material in the central optic portion.

3. Use of the formulation developed for hydrophobic intraocular lenses according to claim 1, in the intraocular lenses whose central optic portion and peripheral non-optic portion are made of the same polymeric material.

4. Use of the formulation developed for hydrophobic intraocular lenses according to claim 1, in contact lenses, keratoprostheses, capsular bag extension rings, corneal interiors, corneal rings.

5. A production method of a formulation developed for hydrophobic intraocular lenses according to claim 1, **characterized by**
- mixing phenylethyl methacrylate (PEMA), butyl acrylate (BA), trifluoroethyl methacrylate (TFEMA), ethylene glycoldimethacrylate (EGDMA), 2- (2'-Hydroxy-5'-methacryloxy ethylphenyl) -2H-benzotriazole (UV absorber) and azobisisobutyronitrile (AIBN).
- pouring the mixture into a two-piece mold cavity made of polypropylene and closing the mold without air entrapment.
- performing curing process by placing the closed mold in a room equipped with UV lamps,
- polymerization as a result of curing process,
- obtaining a polymer material comprising a formulation developed for hydrophobic intraocular lenses which is the final product.

6. Production method of a formulation developed for hydrophobic intraocular lenses according to claim 5, **characterized in that** the curing is carried out in a thermal oven at 90°C to 100 C for 1-2 hours.

## Patentansprüche

1. Eine für hydrophobe Intraokularlinsen entwickelte Formulierung, die in der ophthalmischen Vorrichtung verwendet werden kann, die durch einen Schnitt in das Auge bei der Behandlung von Katarakten eingeführt wird, und die Alkylacrylat, Fluoracrylat und Phenylmethacrylat umfasst, **dadurch gekennzeichnet, dass** sie in Masse 46. 0 % Phenylethylmethacrylat (PEMA), 40,0 % Butylacrylat (BA), 10,0 % Trifluorethylmethacrylat (TFEMA), 3,0 % Ethylenglycoldimethacrylat (EGDMA) als Vernetzer, 0,5 % 2- (2'-Hydroxy- 5'-methacryloxyethylphenyl) -2H-Benzotriazol (UV-Absorber) und 0,5 % Azobisisobutyronitril (AIBN).

2. Verwendung der für hydrophobe Intraokularlinsen entwickelten Formulierung nach Anspruch 1 in Intraokularlinsen, deren zentraler optischer Teil aus einem polymeren Material besteht und deren peripherer nicht-optischer Teil aus einem zweiten polymeren Material besteht, das sich von dem polymeren Material im zentralen optischen Teil unterscheidet.

3. Verwendung der für hydrophobe Intraokularlinsen entwickelten Formulierung nach Anspruch 1 in Intraokularlinsen, deren zentraler optischer Teil und peripherer nicht-optischer Teil aus demselben polymeren Material hergestellt sind.

4. Verwendung der für hydrophobe Intraokularlinsen entwickelten Formulierung nach Anspruch 1 in Kontaktlinsen, Keratoprothesen, Kapselsackverlängerungsringen, Hornhautinnenseiten, Hornhautringen.

5. Verfahren zur Herstellung einer für hydrophobe Intraokularlinsen entwickelten Formulierung nach Anspruch 1, **gekennzeichnet durch**,
- Mischen von Phenylethylmethacrylat (PEMA), Butylacrylat (BA), Trifluorethylmethacrylat (TFEMA), Ethylenglycoldimethacrylat (EGDMA), 2-(2'-Hydroxy-5'-methacryloxyethylphenyl)-2H-benzotriazol (UV-Absorber) und Azobisisobutyronitril (AIBN).
- Gießen der Mischung in eine zweiteilige Form aus Polypropylen und Schließen der Form ohne Lufteinschluss.
- Durchführung des Aushärtungsprozesses, indem die geschlossene Form in einen mit UV-Lampen ausgestatteten Raum gestellt wird,
- Polymerisation als Ergebnis des Aushärtungsprozesses,
- Gewinnung eines Polymermaterials, das eine für hydrophobe Intraokularlinsen entwickelte Formulierung umfasst, die das Endprodukt darstellt.

6. Verfahren zur Herstellung einer für hydrophobe Intraokularlinsen entwickelten Formulierung nach Anspruch 5, **dadurch gekennzeichnet, dass** die Aushärtung in einem Wärmeschrank bei 90°C bis 100 C für 1 bis 2 Stunden durchgeführt wird.

## Revendications

1. Une formulation développée pour les lentilles intraoculaires hydrophobes, qui peut être utilisée dans le dispositif ophtalmique inséré dans l'œil par une incision dans le traitement de la cataracte, comprenant de l'acrylate d'alkyle, de l'acrylate de fluor et du méthacrylate de phényle, **caractérisée en ce qu'**elle comprend en masse : 46,0 % de méthacrylate de phényléthyle (PEMA), 40,0 % d'acrylate de butyle (BA), 10,0 % de méthacrylate de trifluoroéthyle (TFEMA), 3,0 % d'éthylèneglycoldiméthacrylate (EGDMA) comme agent de réticulation, 0,5% de 2-(2'-Hydroxy-5'-méthacryloxyéthylphényl) -2H-benzotriazole (absorbeur d'UV) et 0,5 % d'azobisisobutyronitrile (AIBN).

2. Utilisation de la formulation développée pour les lentilles intraoculaires hydrophobes selon la revendication 1, dans des lentilles intraoculaires dont la partie optique centrale est constituée d'un matériau polymère et dont la partie périphérique non optique est constituée d'un deuxième matériau polymère différent du matériau polymère de la partie optique centrale.

3. Utilisation de la formulation développée pour les lentilles intraoculaires hydrophobes selon la revendication 1, dans les lentilles intraoculaires dont la partie optique centrale et la partie non optique périphérique sont constituées du même matériau polymère.

4. Utilisation de la formulation développée pour les lentilles intraoculaires hydrophobes selon la revendication 1, dans les lentilles de contact, les kératoprothèses, les anneaux d'extension du sac capsulaire, les intérieurs cornéens, les anneaux cornéens.

5. Procédé de fabrication d'une formulation développée pour des lentilles intraoculaires hydrophobes selon la revendication 1, **caractérisé par**
- le mélange de méthacrylate de phényléthyle (PEMA), d'acrylate de butyle (BA), de méthacrylate de trifluoroéthyle (TFEMA), de diméthacrylate d'éthylène glycol (EGDMA), de 2-(2'-hydroxy-5'-méthacryloxyéthylphényl) -2H-benzotriazole (absorbeur d'UV) et d'azobisisobutyronitrile (AIBN).
- le versement du mélange dans une cavité de moule en deux parties en polypropylène et fermeture du moule sans emprisonnement d'air,
- réalisation du processus de durcissement en plaçant le moule fermé dans une pièce équipée de lampes UV,
- la polymérisation résultant du processus de durcissement,
- l'obtention d'un matériau polymère comprenant une formulation développée pour les lentilles intraoculaires hydrophobes, qui constitue le produit final.

6. Procédé de fabrication d'une formulation développée pour des lentilles intraoculaires hydrophobes selon la revendication 5, **caractérisé en ce que** le durcissement est effectué dans un four thermique à une température comprise entre 90 °C et 100 °C pendant 1 à 2 heures.
